(19)

Europäisches Patentamt

European Patent Office

Office européen des brevets

(11) **EP 4 023 241 A1**

(12) **EUROPEAN PATENT APPLICATION**
published in accordance with Art. 153(4) EPC

(43) Date of publication:
06.07.2022 Bulletin 2022/27

(21) Application number: 20858869.9

(22) Date of filing: 28.08.2020

(51) International Patent Classification (IPC):
*A61K 38/17* (2006.01)      *A61P 7/00* (2006.01)
*A61P 25/00* (2006.01)

(52) Cooperative Patent Classification (CPC):
A61P 25/00; A61K 9/0019; A61K 38/1709;
A61K 45/06; A61P 9/10

(86) International application number:
PCT/CN2020/112217

(87) International publication number:
WO 2021/037230 (04.03.2021 Gazette 2021/09)

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR
Designated Extension States:
BA ME
Designated Validation States:
KH MA MD TN

(30) Priority: 30.08.2019  CN 201910815496

(71) Applicant: Suzhou Yabao Pharmaceutical R&D
Co., Ltd.
Suzhou, Jiangsu 215000 (CN)

(72) Inventors:
• YANG, Lei
  Suzhou, Jiangsu 215000 (CN)
• LIAN, Guoning
  Suzhou, Jiangsu 215000 (CN)

• GAO, Xiaoping
  Suzhou, Jiangsu 215000 (CN)
• ZHU, Lin
  Suzhou, Jiangsu 215000 (CN)
• WANG, Chuan
  Suzhou, Jiangsu 215000 (CN)
• ZHU, Taotao
  Suzhou, Jiangsu 215000 (CN)
• WANG, Xingxing
  Suzhou, Jiangsu 215000 (CN)
• ZHUO, Lang
  Suzhou, Jiangsu 215000 (CN)

(74) Representative: Gille Hrabal
Partnerschaftsgesellschaft mbB
Patentanwälte
Brucknerstraße 20
40593 Düsseldorf (DE)

(54) **USE OF ANNEXIN A5**

(57) The disclosure relates to the field of biomedicine, in particular to the use of annexin A5. The disclosure provides the use of annexin A5 for preventing and/or treating a disease or condition, wherein the disease or condition is selected from cerebral stroke, neurobehavioral deficit, neurodegenerative disease, nerve injury, excessive activation of immune cells in the brain, blood-brain barrier injury, brain edema, increased calcium content, excitatory toxicity, over-activation of NOS and a disease or condition caused by at least one of the above disease or condition.

Fig. 1

EP 4 023 241 A1

**Description**

This present application claims priority and benefits of Chinese Patent Application No. 201910815496.0, filed before China National Intellectual Property Administration on August 30, 2019 and entitled "Use of Annexin A5 for treating cerebral stroke", the entire content of which is incorporated herein by reference in its entirety.

**FIELD**

The present disclosure relates to the field of biomedicine, in particular to the use of Annexin A5.

**BACKGROUND**

Annexin belongs to calcium-dependent phospholipid binding protein family, which is widely expressed and has many important functions. Some annexins, such as annexin A1, are closely related to tumors and are highly expressed in pancreatic cancer, liver cancer and head and neck tumors. Some annexins, such as Annexin A5 (or A5), have anti-inflammatory and anticoagulant functions. Annexin A5, first found in human placenta in 1970, has a gene located on chromosome 4q26-q28, and 13 exons and 12 introns. Annexin A5 is a calcium-dependent channel protein that binds to the surface of negatively charged phospholipids. Its molecular weight is about 34 kDa. It mainly exists in the cell membrane and endoplasmic reticulum. Its high molecular weight usually prevents it from entering brain tissue. Therefore, little research has been done in the nervous system.

"Cerebral stroke", as an acute cerebrovascular disease, is also known as "stroke" or "cerebrovascular accident" (CVA). It comprises ischemic and hemorrhagic strokes and is caused by sudden rupture of blood vessels in the brain or the failure of blood to flow into the brain due to blockage of blood vessels. The incidence of ischemic stroke is higher than that of hemorrhagic stroke, and accounts for 60%-70% of the total number of cerebral strokes. Clinical studies have shown that many factors can induce cerebral stroke. Since cerebral stroke is characterized by high morbidity, disability, recurrence and mortality, there is still a need to further improve the drug therapy of cerebral stroke by means of a new therapeutic agent.

In addition, the treatment of diseases or conditions such as neurobehavioral deficits, neurodegenerative diseases, nerve injury, over-activation of immune cell in the brain, damage of blood-brain barrier, increase of calcium content, excitotoxicity (excitatory toxicity), over-activation of NOS and so on also need to be further improved. Therefore, there is an urgent need to develop a new therapeutic agent.

Although the structure of annexin A5 has been gradually clear, the research on its function and use still needs to be explored by researchers, developed and confirmed by means of experiments. At the same time, the safety of the protein in prevention and/or treatment of such diseases also needs to be investigated.

**SUMMARY**

In order to improve the above technical problems, the present disclosure provides the use of annexin A5 for preventing and/or treating a disease or condition, wherein the disease or condition is selected from cerebral stroke, neurobehavioral deficit, neurodegenerative disease, nerve injury, excessive activation of immune cells in the brain, blood-brain barrier injury, brain edema, increased calcium content, excitatory toxicity, over-activation of NOS and a disease or condition caused by at least one of the above disease or condition.

According to embodiments of the present disclosure, the present disclosure provides the use of annexin A5 in the manufacture of a drug, wherein the drug is used for preventing and/or treating the above disease or condition, or a disease or condition caused by at least one of the above disease or condition.

According to embodiments of the present disclosure, said neurobehavioral deficit, neurodegenerative disease, nerve injury, excessive activation of immune cells in the brain, blood-brain barrier injury, brain edema, increased calcium content, excitatory toxicity, over-activation of NOS can be a disease or condition caused by cerebral stroke or another factor.

According to embodiments of the present disclosure, the cerebral stroke is selected from ischemic stroke and hemorrhagic stroke, such as ischemic stroke during acute phase and/or rehabilitation phase, and hemorrhagic stroke during acute phase and/or rehabilitation phase, especially acute ischemic stroke or ischemic stroke during acute phase.

According to embodiments of the present disclosure, the neurobehavioral deficit includes, but are not limited to, limb paralysis and/or limb weakness.

According to embodiments of the present disclosure, the neurodegenerative disease includes acute neurodegenerative disease and/or chronic neurodegenerative disease, such as an acute neurodegenerative disease selected from cerebral ischemia (CI), brain injury (BI), and epilepsy; and a chronic neurodegenerative disease selected from Alzheimer's disease (AD), Parkinson's disease (PD), Huntington's disease (HD), amyotrophic lateral sclerosis (ALS), different types of spinocerebellar ataxia (SCA), Pick's disease, etc.

According to embodiments of the present disclosure, the nerve injury can be selected from brain nerve injury, such as such as a brain nerve injury caused by craniocerebral trauma, cerebral vascular sclerosis, encephalitis, meningitis, demyelinating disease or an other cerebrovascular disease, such as cognitive nerve injury, for example, olfactory nerve injury, visual nerve injury, auditory nerve injury, taste nerve injury, facial nerve injury, such as cognitive nerve injury, for example, olfactory nerve injury, visual nerve injury, auditory nerve injury, taste nerve injury, facial nerve injury, etc.

According to embodiments of the present disclosure, the nerve injury can be selected from a brain nerve injury caused by oxygen/glucose deprivation or excitotoxicity.

According to embodiments of the present disclosure, the neurodegenerative disease, nerve injury or the disease or condition caused thereby can be selected from affective disorder, depression, severe depressive disorder, postpartum depression, depression related to bipolar disorder, Alzheimer's disease, psychosis, Parkinson's disease, Huntington's disease, anxiety, generalized anxiety disorder, social anxiety disorder, obsessive-compulsive disorder panic disorder, panic attack, phobia, social phobia, agoraphobia, urinary incontinence, vomiting, partial response, treatment resistant depression, cognitive impairment, memory decline, ADHD (attention deficit and hyperactivity disorder), melancholia, and PTSD (post-traumatic stress disorder).

According to embodiments of the present disclosure, the term "immune cells in the brain" in the over-activation of immune cells in the brain includes immunocompetent cell, especially microglia of the central nervous system (CNS).

According to embodiments of the present disclosure, the blood-brain barrier injury includes an injury, damage or obstacle of a barrier between plasma and brain cells and/or a barrier between plasma and cerebrospinal fluid, such as a barrier injury between blood plasma and brain cells formed by capillary wall and glial cells and/or a barrier injury between blood plasma and cerebrospinal fluid formed by choroid plexus. The blood-brain barrier injury can be caused by the above diseases or conditions, or by craniocerebral injury (such as craniocerebral trauma caused by external force).

According to embodiments of the present disclosure, the brain edema refers to the pathological phenomenon of an increase of brain volume caused by an increasing water content in the brain, which can lead to intracranial hypertension that causes damage to brain tissue. It can be selected from the brain edema caused by brain injury, blood-brain barrier injury, microcirculation disturbance, cerebral hemorrhage, cerebral ischemia, cerebral hypoxia and an other factor.

According to embodiments of the present disclosure, the increased calcium content refers to an increased concentration of calcium ion in brain tissue beyond the normal range of the same.

According to embodiments of the present disclosure, the excitotoxicity is selected from N-methyl-D-aspartic acid (N-methyl-D-aspartate) induced excitotoxicity.

According to embodiments of the present disclosure, the over-activation of NOS refers to the over-synthesis of NO caused by the activation of NOS.

[0022] According to embodiments of the present disclosure, said neurobehavioral deficit, neurodegenerative disease, nerve injury, excessive activation of immune cells in the brain, blood-brain barrier injury, brain edema, increased calcium content, excitatory toxicity, over-activation of NOS can be caused by cerebral stroke. Accordingly, where the use of annexin A5 or the drug prepared therefrom involves the prevention or treatment of cerebral stroke, said use can be further characterized by the following technical features.

According to embodiments of the present disclosure, the cerebral stroke is ischemic stroke, particularly acute ischemic stroke or ischemic stroke during acute phase. Annexin A5 can improve the neurobehavioral deficit caused by ischemic stroke, such as limb paralysis, or reduce the area of cerebral infarction, so as to achieve the goal of treating ischemic stroke.

According to embodiments of the present disclosure, the cerebral stroke involves over-activation of immune cell in the brain. Annexin A5 can be used to inhibit the over-activation of immune cell in the brain, thereby alleviating the nerve injury caused by the over-activation of immune cell in the brain, so as to treat cerebral stroke.

According to embodiments of the present disclosure, the cerebral stroke involves intracerebral nerve injury. Annexin A5 can play a neuroprotective role in the brain, thereby repairing nerve damage in the brain, so as to treat cerebral stroke.

According to embodiments of the present disclosure, the cerebral stroke involves a damaged blood-brain barrier. Annexin A5 can repair the damaged blood-brain barrier and avoid further brain damage, so as to treat cerebral stroke.

According to embodiments of the present disclosure, wherein the immune cell comprises microglia. Over-activation of microglia can release a large number of neurotoxic factors, leading to a neurodegenerative disease. Annexin A5 can inhibit the over-activation of microglia.

According to embodiments of the present disclosure, wherein the nerve injury is intracerebral nerve injury caused by oxygen/glucose deprivation. Annexin A5 can alleviate the intracerebral nerve injury caused by oxygen/glucose deprivation. Hypoxia or hypoglycemia in the brain can induce nerve injury. Annexin A5 can alleviate the nerve injury caused by oxygen and glycemia reduction, so as to treat cerebral stroke.

According to embodiments of the present disclosure, wherein the nerve injury is intracerebral nerve injury caused by excitotoxicity. Excitotoxicity can increase the content of calcium ion in brain tissues and activate NOS, which can lead to a series of physiological or pathological changes such as the synthesis of large amounts of NO, resulting in nerve injury in brain. Annexin A5 can repair the nerve injury, so as to treat cerebral stroke.

According to embodiments of the present disclosure, wherein the excitotoxicity is selected from N-methyl-D-aspartic acid induced excitotoxicity .

According to embodiments of the present disclosure, wherein the annexin A5 can inhibit the over-activation of immune cell in brain.

According to embodiments of the present disclosure, wherein the annexin A5 can repair nerve injury in the brain.

According to embodiments of the present disclosure, wherein the annexin A5 can repair the damaged blood-brain barrier.

Those skilled in the art should recognize that the neurobehavioral deficit, neurodegenerative disease, nerve injury, excessive activation of immune cells in the brain, blood-brain barrier injury, brain edema, increased calcium content, excitatory toxicity, over-activation of NOS can also be caused by another factor other than cerebral stroke, for example, the blood-brain barrier injury caused by craniocerebral trauma.

According to embodiments of the present disclosure, annexin A5 may include, but not limited to, a full-length natural human Annexin A5 polypeptide or its variant. A5 polypeptides can be provided from any source or method, such as natural isolates or recombinant or synthetic sources or appropriate combinations of the above. The polypeptide sequence of annexin A5 can be based on complete or partial natural amino acid sequences or on variants of these complete or partial natural amino acid sequences.

According to embodiments of the present disclosure, wherein the annexin A5 is natural human annexin A5. According to embodiments of the present disclosure, the annexin A5 is a recombinant human annexin A5 expressed in a prokaryotic expression system. Therefore, recombinant human annexin A5 can be quickly and efficiently obtained by the prokaryotic expression system, which is not expensive. Moreover, it has been proved that it has no immunotoxicity and very low immunogenicity. Moreover, the tolerant dose of A5 protein in rats and cynomolgus monkeys is above 4500 $\mu$g/kg, and even above 9000 $\mu$g/kg in rats. It is safe. Even in high dosage, it will not bring about the risk of drug use, this is of great therapeutic significance in treating cerebral stroke which is acute cerebrovascular disease. In some embodiments of the disclosure, the amino acid sequence of the annexin A5 expressed by the prokaryotic expression system is shown as SEQ ID NO:1. The amino acid sequence shown in SEQ ID NO:1 is identical with that of natural human annexin A5. The difference is that the N-terminal amino acid of the recombinant human annexin A5 is alanine (A), while the N-terminal amino acid of natural annexin A5 is acetylated alanine. It can be obtained efficiently and rapidly, and has been proved to be very safe, and will not cause risk of drug use even at high doses. It is of great value in the treatment of stroke.

Moreover, it was found that the sequence of annexin A5 was conservative, and it had high homology in different species and played similar or identical roles. These highly homologous or conservative sequences of annexin A5 could be used to treat stroke or to prepare drugs for stroke. For example, in some embodiments of the disclosure, the sequence identity of the annexin A5 is 96% or more compared with natural human annexin A5, or with the sequence shown in SEQ ID NO:1, such as 96.5% or more, 97% or more, 97.5% or more, 98% or more, 98.3% or more, 98.5% or more, 98.8% or more, 99% or more, 99.3% or more and 99.5% or more. According to some embodiments of the present disclosure, the annexin A5 comprises one conservative amino acid substitution, or two conservative amino acids substitution, or three conservative amino acids substitution, or four conservative amino acids substitution or five conservative amino acids substitution as compared with SEQ ID NO: 1. These highly homologous or conservative amino acid-substituted annexin A5 can be synthesized by artificial design, and can also be isolated or synthesized from natural annexin A5.

According to embodiments of the present disclosure, the drug can present in a form of formulation, such as injection. The content of Annexin A5 in an unit dosage form can be 0.025mg-250mg. Considering the daily dose of Annexin A5 of about 0.05mg-500mg, during the preparation of the drug, the content of Annexin A5 in unit dosage form can be adjusted to 0.025mg-250mg adaptively, such as 0.1-50mg, 0.1-100mg, 0.1-200mg, or 0.1-250mg, or 1-50mg, 1-100mg, 1-200mg or 1-250mg, preferably 1-100mg, for example 1mg, 10mg, 20mg, 30mg, 40mg, 50mg, 60mg, 70mg, 80mg, 90mg or 100mg. By preparing the annexin A5 formulation, the treatment of the above diseases or conditions can be achieved, and has good safety without toxic and side effects. Drugs in unit dosage form refer to a single dosage form that is designed when a drug is prepared into different dosage forms. For example, if a tablet is designed to be 400mg in size, then the 400mg of drug is in a single dosage form. For another example, where a drug is designed as an injection, and each packaged injection is independently as a single dosage form. Usually, a drug in a unit dosage form is used as a daily dose for one day or as a daily dose for half a day. The content of annexin A5 in these unit dosage forms can fluctuate between 0.025and 250mg, and then be prepared into unit dosage forms with a pharmaceutically acceptable carrier.

The "pharmaceutically acceptable carrier" may include a carrier selected from any and all solvents, dispersants, coatings, antibacterial agents, antifungal agents, isotonic agents and delayed absorbents that are physiologically compatible. Among them, a specific example can be one or more selected from water, brine, phosphate buffer brine, glucose, glycerol, ethanol, etc. In many cases, the pharmaceutically acceptable carrier can also be selected from isotonic agents, such as carbohydrates, polyols (such as mannitol, sorbitol) or sodium chloride. Of course, the pharmaceutically acceptable carrier can also include a small amount of an auxiliary substance, such as wetting agent or emulsifier, preservative or buffer, so as to extend the shelf life or potency of antibodies.

The present disclosure further provides a drug comprising annexin A5, wherein the drug is used for preventing and/or treating a disease or condition selected from cerebral stroke, neurobehavioral deficit, neurodegenerative disease, nerve injury, excessive activation of immune cells in the brain, blood-brain barrier injury, brain edema, increased calcium content, excitatory toxicity, over-activation of NOS and a disease or condition caused by at least one of the above disease or condition.

The present disclosure further provides a drug combination, wherein said drug combination comprises annexin A5 and an other drug.

According to embodiments of the present disclosure, the other drug can be selected from drugs other than annexin A5, such as an active ingredient used for preventing and/or treating a disease or condition selected from cerebral stroke, neurobehavioral deficit, neurodegenerative disease, nerve injury, excessive activation of immune cells in the brain, blood-brain barrier injury, brain edema, increased calcium content, excitatory toxicity, over-activation of NOS and a disease or condition caused by at least one of the above disease or condition.

According to embodiments of the present disclosure, the other drug used for preventing and/or treating cerebral stroke can be at least one selected from a thrombolytic drug, an antiplatelet drug, an anticoagulant, a fibrinolytic drug, a plasma expander, a vasodilator or other drug to improve cerebral blood circulation and a neuroprotective agent. By using these drugs in combination, multi-angle and multi-level treatment of stroke can be achieved, so that the stroke can be quickly treated.

The present disclosure further provides a method for preventing and/or treating a disease or condition selected from cerebral stroke, neurobehavioral deficit, neurodegenerative disease, nerve injury, excessive activation of immune cells in the brain, blood-brain barrier injury, brain edema, increased calcium content, excitatory toxicity, over-activation of NOS and a disease or condition caused by at least one of the above disease or condition, wherein the method comprises administrating a therapeutically effective amount of annexin A5 to a patient.

In this context, the term "effective amount" or "therapeutically effective amount" refers to the dosage of the drug that can play the therapeutic role of slowing down or rehabilitation for the disease or condition.

In this context, the term "patient" may be an individual or group suffering from the above disease or condition, or at risk of the above disease or condition, such as a subject. The patient includes human and mammals.

According to some embodiments of the present disclosure, the daily dose of the annexin A5 is 0.05mg-500mg, preferably 2-200mg. Generally, the daily dose of the annexin A5 can be 1mg-500mg/day or 1mg-450mg/day, such as 1mg-400mg/day, 1 mg-350mg/day, 1mg-300mg/day, 1mg-250mg/day, 1mg-200mg/day, etc. Of course, in some patients with mild stroke, the dosage of A5 protein can be less, such as 1mg-100mg/day, or 1mg-80mg/day.

According to the present disclosure, annexin A5 can be administrated either single or multiple times. It can be administered by intravenous injection, intramuscular injection, intraperitoneal injection, subcutaneous injection, intrathecal injection, nasal spray and oral spray. It can also be administrated by short-term rapid administration, including, but not limited to, rapid intravenous injection, intramuscular injection, intraperitoneal injection, subcutaneous injection, intrathecal injection, nasal spray, oral spray, etc. It can be administrated by sustained exposure administration, including but not limited to sustained slow intravenous injection, intramuscular injection, intraperitoneal injection, subcutaneous injection, intrathecal injection, etc. Considering that annexin A5 belongs to protein drugs, it can be preferably to prepared to an injection such as by freeze-dried powder or other injection drugs with specific specifications, so that a rapid treatment and high bioavailability *in vivo* can be achieved.

During treatment, annexin A5 can be used in acute and convalescent stage of the above disease or conditions (such as stroke), secondary preventive administration after stroke, or preventive administration in potential stroke patients with imaging indications but without a clinical symptom.

It should be understood that annexin A5 can be used alone in the treatment of stroke, or in combination with surgery or other drugs. Surgery includes but is not limited to endovascular interventional therapy (such as mechanical thrombectomy, angiogenesis and stent implantation). Other drugs include but are not limited to thrombolytic drugs (such as recombinant tissue plasminogen activator rtPA and urokinase), antiplatelet drugs (such as aspirin, clopidogrel, etc.), anticoagulants, defibrases (such as defibrase and batroxobin), and plasma expanders, drugs for vasodilators, other drugs for improving cerebral blood circulation and neuroprotective agents (e.g. edaravone, etc.). Through the combination of drugs, the stroke can be multi-angle treated, so that it can be quickly treated.

According to some embodiments of the present disclosure, after an effective dose of annexin A5 is administered to a subject, it can inhibit the over-activation of immune cells in brain, such as the over-activation of microglia in brain, thereby indirectly repairing the nerve injury caused by the over-activation of immune cells. At the same time, it can directly repair the brain nerve damage, such as reducing the brain nerve damage caused by oxygen/glucose deprivation, and reducing the brain nerve damage caused by excitotoxicity such as N-methyl-D-aspartic acid. In the process of treating stroke, on the one hand annexin A5 protein can penetrate into the brain through the damaged blood-brain barrier and play a therapeutic role. At the same time, it can repair damaged blood brain barrier to prevent further deterioration of the disease and further achieve the goal of treatment.

In some embodiments of the disclosure, the annexin A5 used is expressed by a prokaryotic expression system, such as an *Escherichia coli* expression system, and the amino acid sequence of annexin A5 is shown in SEQ ID NO: 1.

In the second aspect of the present disclosure, a pharmaceutical composition for treating cerebral stroke is provided, and according to embodiments of the present disclosure, the pharmaceutical composition comprises an annexin A5 or a functional equivalent thereof.

According to embodiments of the present disclosure, wherein the annexin A5 or the functional equivalent thereof is administrated in a unit dosage form comprising 0.025mg-250mg the annexin A5 or the functional equivalent thereof.

According to embodiments of the present disclosure, wherein the annexin A5 or the functional equivalent thereof is administrated in a unit dosage form comprising 1mg-100mg the annexin A5 or the functional equivalent thereof.

Inventors of the present disclosure had found that annexin A5 can be used for treating stroke and repairing nerve injury of brain in a long-term study. It was confirmed by the rat model of ischemia-reperfusion that the annexin A5 could reduce the area of cerebral infarction, improve the symptoms of neurological deficits and promote long-term motor recovery in rats. For example, in a rat model of focal cerebral ischemia established by light irradiation, after the annexin A5 was injected via caudal vein, the neurological deficit symptoms were improved and the area of cerebral infarction decreased significantly. Take another example, in the rat model of hemorrhagic stroke established by intracerebral injection of autologous arterial blood, after the annexin A5 was injected via caudal vein, the neurological deficit symptoms of the rats were significantly improved, and the area of cerebral infarction also decreased.

The inventors also found that the annexin A5 can repair and alleviate stroke-related injuries, so that it can be used to treat stroke diseases.

The inventors also found that in the case of acute injury (stroke, trauma, etc.), when the blood-brain barrier is damaged, the radiolabeled annexin A5 as a developer will be distributed into the brain. After annexin A5 is administrated of, however, it can significantly repair the blood-brain barrier injury, brain nerve injury or the above-mentioned related diseases. In addition, annexin A5 entering into the brain can be used to treat brain diseases and repair brain nerve injury. For example, it can repair brain ischemia or reperfusion injury, so as to prevent or treat the diseases or conditions described above. Surprisingly, while achieving the above excellent treatment effect, the subjects have no risk of bleeding during the treatment, showing that the drug of the present disclosure also has excellent safety.

In this context, "repair" or "alleviate" refers to a way or means for reducing the above brain damage.

## DESCRIPTION OF DRAWINGS

Fig. 1 is a diagram showing the effect of annexin A5 solution on the symptoms of nerve defects according to the following example.

Fig. 2 is a diagram showing the effect of annexin A5 solution on the scope of cerebral infarction according to the following example.

Fig. 3 is a diagram showing the effect of annexin A5 on OGD injury of primary cortical neurons provided according to the following example 4.

Fig. 4 is a diagram showing the protective effect of annexin A5 on NMDA induced excitatory injury of primary cortical neurons in rats according to the following example 5.

Fig. 5 is a diagram showing the detection results of EB content in brain tissue by annexin A5 according to the following example 6.

## DETAILED DESCRIPTION

The following embodiments described by reference to the accompanying drawings are illustrative and are intended to be used to explain the present disclosure rather than to constrain it.

Inventors of the present disclosure had found that the annexin A5, which can repair or alleviate injury, or plays a role in the prevention and/or treatment according to the present disclosure, may be the full length or partial of amino acid sequence of the natural human annexin A5 polypeptide, or may be a variant of the full length or partial amino acid sequence of the natural human annexin A5 polypeptide. Of course, the preparation or acquisition of these natural annexins or variants can be based on any source or method, such as direct separation from naturally occurring substances, artificial synthesis, or any combination of the above methods.

For example, as compared with human natural full-length annexin A5, useful annexin A5 can have at least 97% sequence identity or 98% sequence identity, for example, at least 98.5%, at least 98.8%, or at least 99%, for example, at least 99.3% or at least 99.5%, or at least 99.6%. The amino acid sequences which are homologous to human natural full-length annexin A5 can have differences in one amino acid, two amino acids, three amino acids, four amino acids, or even five amino acids, six amino acids and seven amino acids. The amino acids differences can be conservative amino acids substitution in the natural full-length annexin A5 sequence. "Conservative amino acid substitution" can refer to the substitution of an amino acid by a biologically, chemically or structurally similar residue. Biological similarity refers to the biological activity of annexin A5 is not interrupted by the substitution. Structural similarity means that amino acids have side chains of similar length, such as alanine, glycine or serine, or side chains of similar size. Chemical similarity refers to those amino acids have the same charge or are all hydrophilic or all hydrophobic. For example, hydrophobic residues such as isoleucine, valine, leucine or methionine are substituted for each other. Or use polar amino acids such as arginine instead of lysine, glutamic acid instead of aspartic acid, glutamine instead of aspartic amide, serine instead of threonine and so on.

The above amino acid sequences, which show homology or conservative amino acid substitution, can be synthesized by artificial design and can also exist directly in other species in nature. It was found that the sequence of annexin A5 is conservative and show high homology in different species. The amino acid sequence of SEQ ID NO:1 is the same as that of natural human annexin A5, the difference is that the N-terminal amino acid of SEQ ID NO:1 is alanine (A), while the N-terminal of natural annexin A5 is acetylated alanine. Therefore, the mutated sites and amino acids in different species shown in Table 1 below are compared with SEQ ID NO:1 sequence, which also reflects the difference between the Annexin A5 sequences of different species in nature and the human natural Annexin A5 sequence. Compared with SEQ ID NO:1 sequence, the third amino acid mutated from valine to isoleucine in Gorilla gorillas. For another example, it is also reported in J. Mol. Biol. 223 (3), 683-704 (1992) and J. Mol. Biol. 223 (3), 683-704 (1992), there are variations in natural human annexin, that is, compared with natural annexin A5, the 76th amino acid mutates from glutamic acid to glutamine or from glutamic acid to glycine. Table 1 below lists the mutation sites and corresponding amino acids in some species compared with SEQ ID NO:1 sequence. These sequences can be obtained by NCBI Access Number in NCBI or directly in relevant references.

Table 1: Compared with SEQ ID NO:1, the mutation sites and amino acids existed in different species

| Species | The corresponding loci in SEQ ID NO:1 | Variation | NCBI Access Number or reference |
|---|---|---|---|
| Gorilla | 3 | V→I | XP_004040389.1 |
| human | 76 | E→Q | J. Mol. Biol. 223 (3), 683-704 (1992) |
| human | 76 | E→G | J. Mol. Biol. 223 (3), 683-704 (1992) |
| human | 134 | S→L | CAG38759.1 |
| Lipotes vexillifer | 53-54 | SA→AV | XP_007464903.1 |
| | 207 | K→R | |
| Orcinus orca | 53-54 | SA→AV | XP_004265141.1 |
| | 207 | K→R | |
| | 318 | D→E | |
| Tursiops truncatus | 53-54 | SA→AV | XP_004321032.1 |
| | 207 | K→R | |
| | 272 | M→V | |
| Lagenorhynchus obliquidens | 53-54 | SA→AV | XP_026959424.1 |
| | 207 | K→R | |
| | 272 | M→V | |
| | 88 | R→Q | |

(continued)

| Species | The corresponding loci in SEQ ID NO:1 | Variation | NCBI Access Number or reference |
|---|---|---|---|
| Nomascus leucogenys | 141 | G→E | XP_003271381.1 |
| | 209 | F→L | |
| | 317-318 | ED→GE | |
| Delphinapterus leucas | 1-3 | AQV→SQ A | XP_022451968.1 |
| | 53-54 | SA→AV | |
| | 207 | K→R | |
| Macaca fascicularis | 105 | N→D | NP_001270160.1 |
| | 317-318 | ED→GE | |
| Saimiri boliviensis | 54 | A→E | XP_010345019.1 |
| | 51 | E→K | |
| | 315 | C→CG | |

The embodiments of the present disclosure will be explained with the following examples. It will be understood by those skilled in the art that the following examples are merely used to exemplarily explain and illustrate the present disclosure and should not be regarded as limiting the scope of the present disclosure. Where no specific technology or condition is specified in the following examples, it shall be carried out in accordance with the technology or condition described in the literature in the field or in accordance with the product specification. The reagent or instrument used does not specify the manufacturer, and is a conventional product that can be purchased from the market.

Example 1

Annexin A5 (hereinafter also referred to as A5 protein) used in this example and the following examples comes from human full-length annexin. By constructing recombinant vector, A5 protein was expressed in E. coli. After sequencing, the amino acid sequence of A5 protein was as follows:

AQVLRGTVTDFPGFDERADAETLRKAMKGLGTDEESILTLLTSRSNAQRQEISAAFK

TLFGRDLLDDLKSELTGKFEKLIVALMKPSRLYDAYELKHALKGAGTNEKVLTEIIASRTPE

ELRAIKQVYEEEYGSSLEDDVVGDTSGYYQRMLVVLLQANRDPDAGIDEAQVEQDAQA

LFQAGELKWGTDEEKFITIFGTRSVSHLRKVFDKYMTISGFQIEETIDRETSGNLEQLLLAV

VKSIRSIPAYLAETLYYAMKGAGTDDHTLIRVMVSRSEIDLFNIRKEFRKNFATSLYSMIKG

DTSGDYKKALLLLCGEDD （SEQ ID NO:1） .

As shown in SEQ ID NO:1, annexin A5, expressed by E. coli expression system, contains 319 amino acids. The cDNA sequence is encoded by 320 amino acid codons, and methionine at N-terminal is removed during expression. The amino acid sequence of SEQ ID NO:1 is identical to that of natural human annexin A5, except that the N-terminal amino acid of annexin A5 expressed by this system is alanine (A), while the N-terminal amino acid of natural human annexin A5 is acetylated alanine.
The annexin A5 obtained from example 1 is then used to perform the following experiments.

**Example 2**

Refer to the literature *Behavioral deficits and recovery following transient focal cerebral ischemia in rats: glutamatergic and GABAergic receptor densities* (Jolkkonen J, Gallagher NP, Zilles K, Sivenius J. Behav Brain Res 2003;

138:187-200*) and literature *Reversible middle cerebral occlusion without craniery in rats. (*Longa EZ, Weinstein PR, Carlson S, Cummins R.Stroke 1989;20:84-91), the cerebral ischemia-reperfusion model of middle cerebral artery occlusion (MCAO) was established by internal carotid artery thread embolization. A5 (A5 protein) 0.1mg/kg group, A5 1.0mg/kg and A5 10.0mg/kg group were set up respectively. Edaravone was injected intravenously (6.0mg/kg) as a positive control group. The model group and sham operation group were set up separately. The model group and sham operation group were given 0.9% sodium chloride injection of the same volume. Each dose group of A5 protein injection can be given once at the time of reperfusion, once again at 1 hour after reperfusion, and the other groups can be given once immediately after reperfusion. After 24 hours of cerebral ischemia, the symptoms of neurobehavioral deficits and the area of cerebral infarction were evaluated.

**1. Experimental material**

(1) experimental animal

Sprague Dawley (SD) rat, SPF grade, male, aged 6-8 weeks or so, weighing 250-280 g。

Animals will be excluded in the course of the experiment in situations as follows: a) death during anesthesia; b) death during cerebral ischemia; c) death after reperfusion until the evaluation of the index; d) detection of basilar hemorrhage after brain extraction; e) right cerebral infarction-free area after TTC staining.

(2) reagents

The solution of A5 protein is prepared by diluting the A5 protein prepared in example 1 with 0.9% sodium chloride solution.

Control substance: Edaravone Injection (purchased from Nanjing Xiansheng Dongyuan Pharmaceutical Co., Ltd.) was diluted with 0.9% sodium chloride injection in the proportion of 1:1 as a positive control.

**2. Experimental design**

(1) dose design

Table 2: Dose Table for Pharmacodynamic Test of A5 Protein

| Group | Test | Dosage (mg/kg) | Preparation concentration (mg/mL) | Dosage volume (mL/kg) | Number of main test animals |
|---|---|---|---|---|---|
| A5 0.1 mg/kg group | A5 protein | 0.1 | 0.02 | 5 | -20 |
| A5 1.0 mg/kg group | A5 protein | 1.0 | 0.2 | 5 | -20 |
| A5 10.0 mg/kg group | A5 protein | 10.0 | 2 | 5 | -20 |
| Edaravone | Edaravone injection | 6.0 | 1.0 | 6 | -20 |
| model group | 0.9% sodium chloride injection | - | - | 5 | ~20 |
| sham operation group | 0.9% sodium chloride injection | - | - | 5 | ~10 |

(2) Drug administration

The animals in each group were injected intravenously through the caudal vein at a rate of about 1.5-2.5 ml / min.

After 24 hours of cerebral ischemia, the symptoms of neurobehavioral deficits and the area of cerebral infarction were evaluated.

**3 evaluation method**

(1) neurological deficit symptom score

According to the method described in *Rat middle cerebral artery occlusion, evaluation of the model and development of a neurologic examination(*Bederson JB, Pitts LH, Tsuji M, Nishimura MC, Davis RL, Bartkowski H. 1986.Stroke 17: 472-476*)*, the neurological deficit symptoms of animals were evaluated by the improved Bederson 5-point system after 24 hours of ischemia.

(2) Measurement of the degree of cerebral infarction

According to *Evaluation of 2,3,5-triphenyltetrazolium chloride as a stain for detection and quantification of experimental cerebral infarction in rats(*Bederson JB, Pitts LH, Germano SM, Nishimura MC, Davis RL, Bartkowski HM. Stroke 1986; 17:1304-8) and *Quantification of infarct size on focal cerebral ischemia model of rats using a simple and economical method(*Yang Y, Shuaib A, Li Q. JNeurosci Methods 1998;84:9-16*)*, determine the degree of cerebral infarction by TTC staining.

Calculation of infarct area: the photos were processed by Image J software, and the corresponding area of left brain and non-infarct area of right brain were calculated according to the formula, and the percentage of infarct area was calculated.

Method of Infarct volume calculation:

$$V=t\ (A1+A2+A3+\ldots\ldots\ldots+An)$$

t is the slice thickness; A is the infarct area.

$$\%I=(V_C-V_L)/V_C\times100\%$$

%I is the percentage of infarct area, Vc is the volume of control side (left hemisphere); $V_L$ is the volume of non-infarct area of infarct side (right hemisphere).

(3) evaluation index

The range of cerebral infarction and neurobehavioral score were used as the main evaluation indexes, and the clinical manifestations of animals were observed.

The quantitative data were expressed as mean ± standard error. Each efficacy index was analyzed by one-way ANOVA with GraphPad prism (6.01). After significant variance test, Fisher's LSD test was used to test the differences between groups. P < 0.05 was defined as significant difference.

**4 experimental result**

(1) the effect of A5 protein on the symptoms of nerve deficiency

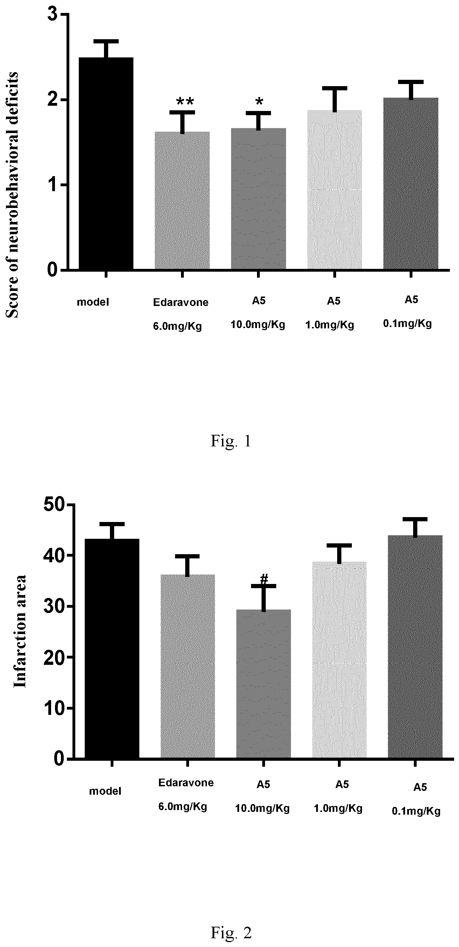

With one-way analysis of variance, there was no statistical difference between the groups (f (4.69) = 2.305, P = 0.0669). However, t-test was carried out between each group and model group respectively. Edaravone 6.0mg/kg group (P < 0.01) and A5 10.0mg/kg group (P < 0.05) could significantly improve the neurobehavioral deficit symptoms of ischemic animals; A5 1.0mg/kg group and A5 0.1mg/kg group had the tendency to improve the neurobehavioral deficit symptoms of ischemic animals, but there was no statistical difference. The influence of A5 injection on neurobehavioral deficits is showed in Table 3 and figure 1.

Table 3: The effect of A5 protein on acute cerebral ischemia-reperfusion injury in rats (Mean±SEM)

| Group | The number of rats | Neurological deficit symptom score | Scope of cerebral infarction (%) |
|---|---|---|---|
| sham operation group | 10 | 0 | 0 |

(continued)

| Group | The number of rats | Neurological deficit symptom score | Scope of cerebral infarction (%) |
|---|---|---|---|
| model group | 15 | 2.47±0.22 | 42.53±3.27 |
| Edaravone 6.0mg/kg | 15 | 1.60±0.25** | 35.93±3.94 |
| A5 10.0mg/kg | 14 | 1.64±0.20* | 28.97±5.08[#] |
| A5 1.0mg/kg | 14 | 1.86±0.27 | 38.37±3.61 |
| A5 0.1mg/kg | 16 | 2.00±0.20 | 43.61±3.60 |

Compared with the model group, * $P < 0.05$, ** $P < 0.01$, [#] $P < 0.05$

(2) Effect of A5 protein on the Scope of Cerebral Infarction

With one-way analysis of variance, there was no statistical difference between the groups ($F_{(4.69)}$ = 2.240, P = 0.0735). However, t-test was carried out between each group and the model group respectively. A510.0mg/ kg group (P < 0.05) could significantly reduce the cerebral infarct area of model animals; edaravone 6.0mg/kg group and A5 1.0mg/kg group had the trend of reducing the cerebral infarct area of model animals, but there was no statistical difference; A5 0.1mg/kg group had no effect on reducing the cerebral infarct area of model animals. The influence of A5 protein on the area of cerebral infarction is shown in Table 3 and Figure 2.
In this experiment, the difference in the scope of cerebral infarction in the model group was basically within 1/3 of the average (mean ± SD, 42.53 ± 12.65), and the model availability rate was 69.81% (excluding the sham operated group); the experimental system was reliable and could be used for efficacy evaluation.
Compared with the model group, A5 10.0mg/kg group can significantly reduce the cerebral infarction area of the model rats (P < 0.05); edaravone 6.0mg/kg, A5 1.0mg/kg group has the trend of reducing the cerebral infarction area of the model rats, but there is no statistical difference; A5 0.1mg/kg has no effect on reducing the cerebral infarction area of the model rats. Compared with the model group, edaravone 6.0mg/kg and A5 10.0mg/kg can significantly improve the neurobehavioral deficit symptoms of ischemic animals (P < 0.01, P < 0.05); the other groups do not show significant improvement in this index.
In this experiment, A5 10.0mg/kg group can significantly reduce the area of cerebral infarction in rats with ischemia, and improve the symptoms of neurobehavioral deficits in rats with ischemia. There was no bleeding risk in the experimental group treated with A5 protein.

**Example 3**

Microglia plays an important role in the inflammatory process of the central nervous system. The moderate activation of microglia can protect neurons, but the over activated microglia will release a large number of neurotoxic factors, such as carbon monoxide, which will lead to the occurrence of neurodegenerative diseases. Lipopolysaccharide (LPS) can activate microglia, which leads to the over activation of microglia and the damage of neurons. A5 was used to observe the effect of LPS on the over activation of microglia.
During the experiment, LPS was used to stimulate the primary purified microglia as the control group, and A5 protein was added as the experimental group. For the above treatment, MTT method was used to detect cell viability, and cellular immunochemistry method was used to observe cell morphological changes.
The results showed that LPS could activate the primary microglia, but not the cell viability. A5 protein can inhibit the over activation of microglia induced by LPS. Therefore, A5 protein can be used to inhibit the over activation of immune cells in the brain, such as microglia.

**Example 4**

Oxygen glucose deprivation (OGD) model is a stimulation model simulating ischemia / hypoxia at the cell level. By changing the cell culture conditions, such as putting the cells into the hypoxia box or into the sugar free medium, the damage of cells under the condition of ischemia / hypoxia is simulated.
OGD model was established with the primary cortical neurons of rat, wherein the test concentrations of A5 protein were 0, 41.15, 123.5, 370.4, 1111, 3333 or 10000 nM respectively. The cells in the culture plate were washed twice with PBS, and then the glucose-free DMEM exchanged with 95% $N_2$ + 5% $CO_2$ for 30 minutes was added. Then the cells were quickly placed in 37 °C, 94% $N_2$ + 1% $O_2$ + 5% $CO_2$ hypoxia incubator. After 8 hours of anoxic culture, the corresponding DMEM medium (i.e. DMEM medium containing A5) was added, and then put the plate into a 37 °C 5% $CO_2$ cell incubator for 24 hours of reoxygenation. After 24 hours of culture, morphological changes were observed under inverted microscope, cell survival rate was detected by CCK-8 colorimetry and apoptosis rate was detected by flow cytometry. At the same time, the treatment group of normoxia and normal glucose was set as the control group.

The calculation formula of relative cell viability was as follows:

$$\text{Relative cell viability (\%)} = (\text{administration group - background group}) / (\text{control group - background group}) \times 100\%.$$

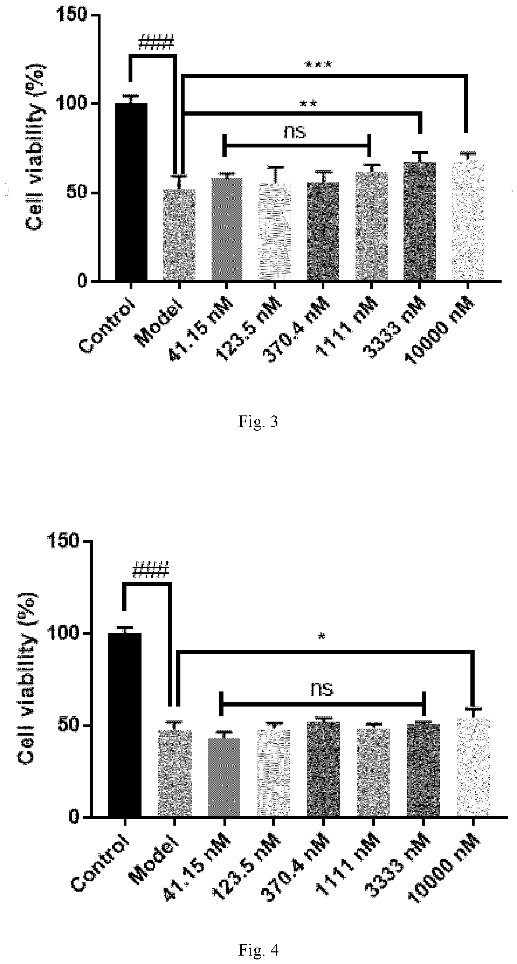

Compared with the model group, A5 protein showed no protective effect on neuronal injury caused by OGD in the concentration range of 41.15-1111 nm (P > 0.05), while significantly reduced the activity after neuronal injury caused by OGD at the concentration of 3333-10000 nm (P < 0.05, Fig. 3).

The experimental results showed that A5 protein could reduce the damage of neurons caused by oxygen glucose deprivation at the concentration of 3333-10000 nm and thus has neuroprotective effect.

**Example 5**

Example 5 studied the neuroprotective effect of A5 protein on NMDA (N-methyl-D-aspartic acid) - induced excitotoxic damage on cortical neurons in rats. NMDA can activate NMDA receptor to increase $Ca^{2+}$ content in brain tissue, and NOS is activated in a large amount, which leads to a series of physiological or pathological changes such as the synthesis of a large number of NO, causing damage to neurons in brain. In order to observe the neuroprotective effect of A5 protein on neuronal damage induced by excitotoxicity, the neuron was pretreated with A5 protein.

In the experiment, 17-day embryonic SD rats were selected and the cortical neurons were cultured. Then the neurons were divided into control group, NMDA group and A5 pretreatment group. The neurons in NMDA group were added with NMDA for exposure, and the neurons in A5 pretreatment group were incubated with A5 protein for a period of time, and then added with NMDA. For different treatment groups, trypan blue staining was used to evaluate cell viability, TUNEL staining was used to detect apoptosis cells and immunofluorescence cytochemistry was used to detect neuron morphology.

The calculation formula of relative cell viability was as follows:

$$\text{Relative cell viability (\%)} = (\text{administration group - background group}) / (\text{control group - background group}) \times 100\%.$$

As shown in Figure 4, NMDA at the concentration of 100 $\mu$M could induce the decrease of cell activity of primary cortical neurons in rats (P < 0.001, compared with normal control group), indicating that the model was established successfully. A5 protein did not significantly reduce the excitatory injury of primary cortical neurons induced by NMDA in the range of detection concentration 41.15-3333 nM (P > 0.05, compared with model group). However, A5 protein showed significant reduction of excitatory injury of primary cortical neurons induced by NMDA at the concentration of 10000 nM (P < 0.05, compared with model group).

The results showed that compared with NMDA group, A5 pretreatment group could reduce the damage of excitatory toxicity to neurons at the concentration of 10000 nM.

**Example 6**

Blood brain barrier is composed of three components: brain microvascular endothelial cells, astrocytes and basement membrane. The damage of blood-brain barrier is one of the important pathologies of stroke.

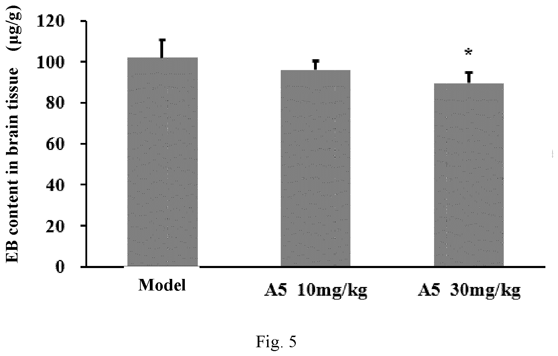

In the experiment, the blood-brain barrier model was established by co-culturing spontaneously transformed endothelial cell line and purified rat astrocytes. The blood-brain barrier (BBB) model *in vitro* was injured after stimulation, and then treated with A5 protein. The integrity of blood-brain barrier was evaluated by using the rat brain injury model caused by craniocerebral strike. A5 10 mg / kg group and A5 30 mg / kg group and a model group were set up respectively. The rats in each A5 protein group were administrated twice via caudal vein injection at 1 hour and 2 hours after modeling respectively. 23 hours after modeling, the rats were anesthetized, and then 2% EB (Evans blue) dye solution was injected through the caudal vein at a dose of 4 mL/kg. The dye was circulated in the rats for 1 hour before death. The heart was perfused with saline through the rat left ventricle until colorless perfusion was obtained. After death, the brain was removed, weighed and homogenized in formamide (3 μL/mg), and then incubated at room temperature for 48 hours. After centrifugation, the supernatant was collected and measured for the optical density at 625 nm to determine the relative amount of EB dye. The results are shown in Table 4 and figure 5:

Table 4 Effect of A5 protein on blood-brain barrier injury induced by craniocerebral injury in rats (mean ± SD)

| Group | Sample size (PCs.) | EB content in brain tissue (μg/g) |
|---|---|---|
| Model group | 5 | 101.87±8.63 |
| A5 10 mg/kg | 5 | 96.08 ± 4.33 |
| A5 30 mg/kg | 5 | 89.36 ± 5.51* |
| Compared with the model group, * P < 0.05. | | |

The results showed that as compared with the model group, there was no significant difference in EB content in brain tissue of A5 10 mg/kg group (P > 0.05), while EB content in brain tissue of A5 30 mg / kg group decreased significantly (P < 0.05). In this experiment, A5 30 mg/kg group could repair the blood-brain barrier injury caused by craniocerebral injury in rats.

The experimental results show that after stimulation, the established blood-brain barrier will be significantly damaged, and A5 can repair the damaged blood-brain barrier.

**Example 7: immunotoxicity and immunogenicity study (repeated dose toxicity study with SD rats and cynomolgus monkeys)**

**1. Toxicity study with SD rats**

SD rats were given A5 protein intravenously for 28 days, and a recovery period of 4 weeks was set up to observe the toxic reaction and severity, main toxic target organs and reversible degree of damage. The dosage was 0 (blank adjuvant), 30, 150 and 750 μg / kg respectively. Blood was sampled to detect immunotoxicity (CD4 [+], CD8 [+] T cells) and immunogenicity. The experiment was divided into four groups, and there were eight rats in each group. The samples were collected at four time points, that is, each rat was sampled for serum at four time points.

The results of immunotoxicity showed that compared with control group, there was no significant difference in the proportion and ratio of peripheral blood T-lymphocyte subsets (CD4[+], CD8 [+] T cell test values) of each dose group.

The immunogenicity results are shown in Table 4. The number of samples in each group in Table 4 represents 8 rats in each group, and each rat is sampled at four time points, with a total of 32 samples in each group; the number of positive samples is the number of positive samples detected in each group.

Table 5: Anti-drug antibody data of A5 protein in rat serum

| Group | Control group | 30 μg/kg dose group | 150 μg/kg dose group | 750 μg/kg dose group |
|---|---|---|---|---|
| Dosage | 0 μg/kg | 30 μg/kg | 150 μg/kg | 750 μg/kg |
| Number of samples in each group | 32 | 32 | 32 | 32 |
| Number of positive samples | 0 | 21 | 22 | 20 |
| Positive rate of total samples | 0.0% | 65.6% | | |

It can be seen from the results given in Table 4 that no anti-drug antibody (ADA) was detected in SD rats after intravenous administration of A5 protein blank adjuvant. After intravenous injection of different doses of A5 protein, the positive rate was 65.6%. There was no significant difference among the dose groups. The positive samples were mainly occurred after the last administration and at the end of the recovery period, which was consistent with the process of the production of drug-resistant antibodies in vivo.

**2. Toxicity study with cynomolgus monkeys**

In this study, A5 protein was injected intravenously into cynomolgus monkeys for 28 days, and a recovery period of 4 weeks was set up to observe the toxic reaction and severity, the main toxic target organs and the reversible degree of damage in cynomolgus monkeys. The dosage was 0 (blank adjuvant), 15, 75 and 375 $\mu$g/kg respectively. Blood was sampled to detect immunogenicity and immunotoxicity (CD4[+], CD8[+] T cells).

The results of immunotoxicity showed that compared with control group, there was no significant difference in the proportion and ratio of peripheral blood T-lymphocyte subsets (CD4 [+], CD8 [+] T-cell measured value) of each dose group.

The immunogenicity results are shown in Table 5. Using the same grouping and processing method as the rats in foregoing paragraphs, the difference is that when sampling, there are two more samples in each group.

Table 6: Data of anti-drug antibody to A5 protein in monkey serum

| Groups | Control | 15 $\mu$g/kg dose group | 75 $\mu$g/kg dose group | 375 $\mu$g/kg dose group |
|---|---|---|---|---|
| Dosage | 0 $\mu$g/kg | 15 $\mu$g/kg | 75 $\mu$g/kg | 375 $\mu$g/kg |
| Number of samples in each group | 34 | 34 | 34 | 34 |
| Number of positive samples | 0 | 7 | 6 | 13 |
| Positive rate of total samples | 0.0% | 25.5% | | |

It is not difficult to see from the results given in Table 5 that ADA was not detected in cynomolgus monkeys after intravenous administration of A5 protein blank adjuvant. After intravenous injection of different doses of A5 protein, the detection rate of the positive samples was 25.5%. The positive samples mainly occurred after 4 weeks of administration and at the end of the recovery period, which was consistent with the process of the production of drug-resistant antibodies in vivo.

The results showed that no immunotoxicity was found in rats and cynomolgus monkeys. In the immunogenicity test, the positive rate of cynomolgus monkeys was significantly lower than that of rats. Considering that A5 protein belongs to human protein, it is expected that its immunogenicity incidence in human body will be significantly reduced.

**Example 8: Study on toxicity of A5 protein in single intravenous injection in SD rats and cynomolgus monkeys**

1. Toxicity of A5 protein in single intravenous injection in SD rats

In this study, A5 protein was injected intravenously once in SD rats to observe the acute toxicity, severity and main toxic target organs. The dosage was 0 (blank adjuvant), 1000, 3000 and 9000 $\mu$g/kg respectively. After the intravenous injection, the animal's posture, gait, reaction, nerve activity, appetite, fur, eyes, ears, mouth, nose, limbs, breath, feces and other clinical symptoms were observed continuously. After that, the recovery from toxic reaction was observed continuously until the 14th day after administration. The animals were dissected and examined for macropathology 15 days after administration. During the experiment, the clinical symptoms of all animals were normal; the weight changes of female and male animals in the administration group were normal; no abnormality was found in all tissues and organs in the macropathological examination. The maximum-tolerated dose (MTD) of single intravenous injection of A5 protein was more than 9000 $\mu$g/kg.

2. Toxicity of A5 protein in single intravenous injection in cynomolgus monkeys

A single intravenous injection of A5 protein was used to observe the acute toxicity and its severity and main toxic target organs in cynomolgus monkeys. The dosage was 0 (blank adjuvant), 500, 1500 and 4500 $\mu$g/kg respectively. After the single intravenous injection, observe the clinical symptoms of the animals (including feces, appearance, respiration, nerve reaction, activity status, etc.) until the 15th day after the administration. After administration, the food intake was measured once a day and the body weight was measured once a week. The body temperature, blood pressure and electrocardiograph (ECG) of the animals were measured before and after administration on the day of administration, and again on the 1st, 7th and 15th day of administration. Hematology (including blood coagulation) and serum biochemical examination were carried out for all animals on the 1st, 7th and 15th day after administration, and urine examination was carried out on the 14th day after administration. On the 15th day after administration, the animals were dissected for macropathological examination, and if necessary, for histopathological examination. Results showed that there were no significant changes in clinical symptoms, injection site, body weight, food intake, body temperature, blood pressure, ECG urinalysis, serum biochemical and pathological examination. There was no significant change in prothrombin time (PT), activated partial thromboplastin time (APTT) and thrombin time (TT) on the 13th day of quarantine period, the 1st day and the 15th day after administration. In this experiment, the maximum-tolerated dose (MTD) of cynomolgus monkeys was more than 4500 μg/kg.

The above results showed that the tolerance dose of A5 protein was more than 4500 μg/kg in both SD rats and cynomolgus monkeys, and even more than 9000 μg/kg in SD rats. The results showed that there was no significant toxicity of A5 protein in rats and monkeys. Therefore, we are reminded that A5 protein is safe for the treatment of stroke, and even in the case of high dosage, it will not or almost will not bring the risk of medication.

In the description of the specification, the terms " embodiment", "some embodiments", "examples", "specific examples", or "some examples" means that the specific features, structures, materials or features described in combination with the embodiment or examples are included in at least one embodiment or example of the disclosure. In this specification, the schematic expression of the above terms need not be directed to the same embodiment or example. Moreover, the specific features, structures, materials or features described may be combined in an appropriate manner in any one or more embodiments or examples. In addition, without contradiction, those skilled in the art can combine and combine different embodiments or examples described in the specification and features of different embodiments or examples.

Although the embodiments of the disclosure have been shown and described above, it can be understood that the above embodiments are exemplary and cannot be understood as limitations of the disclosure. Ordinary technicians in the art can change, modify, replace and transform the above embodiments within the scope of the disclosure.

## Claims

1. Use of annexin A5 for preventing and/or treating a disease or condition, wherein the disease or condition is selected from cerebral stroke, neurobehavioral deficit, neurodegenerative disease, nerve injury, excessive activation of immune cells in the brain, blood-brain barrier injury, brain edema, increased calcium content, excitatory toxicity, over-activation of NOS and a disease or condition caused by at least one of the above disease or condition.

2. The use according to claim 1, wherein said neurobehavioral deficit, neurodegenerative disease, nerve injury, excessive activation of immune cells in the brain, blood-brain barrier injury, brain edema, increased calcium content, excitatory toxicity, over-activation of NOS can be a disease or condition caused by cerebral stroke or another factor.

3. The use according to claim 1 or 2, wherein:

   the cerebral stroke is selected from ischemic stroke and hemorrhagic stroke, such as ischemic stroke during acute phase and/or rehabilitation phase, and hemorrhagic stroke during acute phase and/or rehabilitation phase;
   the neurobehavioral deficit is selected from limb paralysis and/or limb weakness;
   the neurodegenerative disease includes acute neurodegenerative disease and/or chronic neurodegenerative disease, such as an acute neurodegenerative disease selected from cerebral ischemia (CI), brain injury (BI), and epilepsy; and a chronic neurodegenerative disease selected from Alzheimer's disease (AD), Parkinson's disease (PD), Huntington's disease (HD), amyotrophic lateral sclerosis (ALS), different types of spinocerebellar ataxia (SCA), Pick's disease;
   the nerve injury can be selected from a brain nerve injury, such as a brain nerve injury caused by craniocerebral trauma, cerebral vascular sclerosis, encephalitis, meningitis, demyelinating disease and an other cerebrovascular disease, such as cognitive nerve injury, for example, olfactory nerve injury, visual nerve injury, auditory nerve injury, taste nerve injury, facial nerve injury; preferably, the nerve injury can be selected from a brain nerve injury caused by oxygen/glucose deprivation or excitotoxicity;
   the immune cells in the brain include immunocompetent cell, especially microglia of the central nervous system (CNS);
   the blood-brain barrier injury includes an injury, damage or obstacle of a barrier between plasma and brain cells and/or a barrier between plasma and cerebrospinal fluid, such as a barrier injury between blood plasma and brain cells formed by capillary wall and glial cells and/or a barrier injury between blood plasma and cerebrospinal fluid formed by choroid plexus;
   the brain edema is selected from the brain edema caused by brain injury, blood-brain barrier injury, microcirculation disturbance, cerebral hemorrhage, cerebral ischemia, cerebral hypoxia and an other factor;
   the increased calcium content refers to an increased concentration of calcium ion in brain tissue beyond the normal range of the same;
   the excitotoxicity is selected from N-methyl-D-aspartic acid (N-methyl-D-aspartate) induced excitotoxicity;
   the over-activation of NOS refers to the over-synthesis of NO caused by the activation of NOS.

4. The use according to any one of claims 1-3, wherein the neurodegenerative disease, nerve injury or the disease or condition caused thereby can be selected from affective disorder, depression, severe depressive disorder, postpartum depression, depression related to bipolar disorder, Alzheimer's disease, psychosis, Parkinson's disease, Huntington's disease, anxiety, generalized anxiety disorder, social anxiety disorder, obsessive-compulsive disorder panic disorder, panic attack, phobia, social phobia, agoraphobia, urinary incontinence, vomiting, partial response, treatment resistant depression, cognitive impairment, memory decline, ADHD (attention deficit and hyperactivity disorder), melancholia, and PTSD (post-traumatic stress disorder).

5. The use according to any one of claims 1-4, wherein:

the cerebral stroke involves over-activation of immune cell in the brain;
the cerebral stroke involves intracerebral nerve injury;
the cerebral stroke involves a damaged blood-brain barrier;
preferably, the immune cell is selected from microglia;
preferably, the nerve injury is at least one selected from the following: intracerebral nerve injury caused by oxygen/glucose deprivation; intracerebral nerve injury caused by excitotoxicity; intracerebral nerve injury caused by an endogenous substance; preferably, the excitotoxicity is selected from N-methyl-D-aspartic acid induced excitotoxicity;
optionally, the annexin A5 inhibits the over-activation of immune cell in brain;
optionally, the annexin A5 repairs nerve injury in the brain;
optionally, the annexin A5 repairs the damaged blood-brain barrier.

6. The use according to any one of claims 1-5, wherein the annexin A5 is selected from a full-length natural human Annexin A5 polypeptide or its variant, preferably a recombinant human annexin A5 expressed in a prokaryotic expression system.

7. The use according to any one of claims 1-6, wherein the amino acid sequence of the annexin A5 is shown as SEQ ID NO:1.

8. A drug comprising annexin A5, wherein the drug is used for preventing and/or treating a disease or condition selected from cerebral stroke, neurobehavioral deficit, neurodegenerative disease, nerve injury, excessive activation of immune cells in the brain, blood-brain barrier injury, brain edema, increased calcium content, excitatory toxicity, over-activation of NOS and a disease or condition caused by at least one of the above disease or condition.

9. The drug according to claim 8, wherein the drug is preferably selected from an injection; preferably, the content of Annexin A5 in unit dosage form is 0.025mg-250mg, preferably 1-100mg.

10. A drug combination, wherein said drug combination comprises:

(1) annexin A5; and
(2) an other drug;

said other drug is selected from drugs other than annexin A5, such as an active ingredient used for preventing and/or treating a disease or condition selected from cerebral stroke, neurobehavioral deficit, neurodegenerative disease, nerve injury, excessive activation of immune cells in the brain, blood-brain barrier injury, brain edema, increased calcium content, excitatory toxicity, over-activation of NOS and a disease or condition caused by at least one of the above disease or condition;
optionally, the other drug used for preventing and/or treating cerebral stroke is at least one selected from a thrombolytic drug, an antiplatelet drug, an anticoagulant, a fibrinolytic drug, a plasma expander, a vasodilator or other drug to improve cerebral blood circulation and a neuroprotective agent.

Fig. 1

Fig. 2

Fig. 3

Fig. 4

Fig. 5

## INTERNATIONAL SEARCH REPORT

| International application No. |
| --- |
| **PCT/CN2020/112217** |

### A.    CLASSIFICATION OF SUBJECT MATTER

A61K 38/17(2006.01)i;  A61P 7/00(2006.01)i;  A61P 25/00(2006.01)i

According to International Patent Classification (IPC) or to both national classification and IPC

### B.    FIELDS SEARCHED

Minimum documentation searched (classification system followed by classification symbols)

A61K; A61P

Documentation searched other than minimum documentation to the extent that such documents are included in the fields searched

Electronic data base consulted during the international search (name of data base and, where practicable, search terms used)

CNABS, DWPI, CNTXT, WOTXT, USTXT, EPTXT, JPTXT, CNKI, Web of Science, 百度学术, Patentics, 中国生物序列检索系统, NCBI, EBI, STN: applicant, inventor, 膜联蛋白A5, Annexin A5, 脑卒中, 中风, stroke, 神经行为缺陷, neurobehavioral deficit, 神经退行性疾病, neurodegenerative disease, 神经损伤, 脑外伤, 脑血管硬化, 脑溢血, 脑血栓, 脱髓鞘疾病, nerve injury, cerebral trauma, cerebral sclerosis, cerebral hemorrhage, thrombosis, infaction, demyelinating disease, 脑炎, 脑水肿, encephalitis, meningitis, edema, 兴奋性毒性, excitotoxicity, sequence 1

### C.    DOCUMENTS CONSIDERED TO BE RELEVANT

| Category* | Citation of document, with indication, where appropriate, of the relevant passages | Relevant to claim No. |
| --- | --- | --- |
| X | CN 107921068 A (S-BIOMEDICS) 17 April 2018 (2018-04-17)<br>claims 1, 9, 11-13, 16 | 1-6, 8-10 |
| Y | CN 107921068 A (S-BIOMEDICS) 17 April 2018 (2018-04-17)<br>claims 1, 9, 11-13, 16 | 7 |
| X | CN 106714814 A (S-BIOMEDICS) 24 May 2017 (2017-05-24)<br>claims 6, 13, 15-17, 20 | 1-6, 8-10 |
| Y | CN 106714814 A (S-BIOMEDICS) 24 May 2017 (2017-05-24)<br>claims 6, 13, 15-17, 20 | 7 |
| Y | CN 101405017 A (LIND STUART E. et al.) 08 April 2009 (2009-04-08)<br>claims 1, 4, 16-18, description paragraph 18 | 7 |
| A | CN 102105594 A (DANA-FARBER CANCER INSTITUTE, INC.) 22 June 2011 (2011-06-22)<br>entire document | 1-10 |

☑ Further documents are listed in the continuation of Box C.       ☑ See patent family annex.

| * | Special categories of cited documents: | "T" | later document published after the international filing date or priority date and not in conflict with the application but cited to understand the principle or theory underlying the invention |
| --- | --- | --- | --- |
| "A" | document defining the general state of the art which is not considered to be of particular relevance | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
| --- | --- |
| **11 November 2020** | **01 December 2020** |

| Name and mailing address of the ISA/CN | Authorized officer |
| --- | --- |
| **China National Intellectual Property Administration (ISA/CN)**<br>**No. 6, Xitucheng Road, Jimenqiao Haidian District, Beijing 100088**<br>**China** | |
| Facsimile No. **(86-10)62019451** | Telephone No. |

Form PCT/ISA/210 (second sheet) (January 2015)

**INTERNATIONAL SEARCH REPORT**

| International application No. |
|---|
| **PCT/CN2020/112217** |

**C.    DOCUMENTS CONSIDERED TO BE RELEVANT**

| Category* | Citation of document, with indication, where appropriate, of the relevant passages | Relevant to claim No. |
|---|---|---|
| A | WO 2012136819 A1 (ATHERA BIOTECHNOLOGIES AB) 11 October 2012 (2012-10-11) entire document | 1-10 |
| A | VAN GOOR, M.-L.P.J. et al. "The Annexin A5-1C/T polymorphism in ischemic stroke: a case-control study." *J THROMB HAEMOST.*, Vol. 3, 31 December 2005 (2005-12-31), pp. 173-175 | 1-10 |
| A | BAHMANI, P. et al. "Visualization of cell death in mice with focal cerebral ischemia using fluorescent annexin A5, propidium iodide, and TUNEL staining." *JOURNAL OF CEREBRAL BLOOD FLOW & METABOLISM.*, Vol. 31,, 19 January 2011 (2011-01-19), pp. 1311-1320 | 1-10 |

Form PCT/ISA/210 (second sheet) (January 2015)

**INTERNATIONAL SEARCH REPORT**

| International application No. |
|---|
| **PCT/CN2020/112217** |

**Box No. II      Observations where certain claims were found unsearchable (Continuation of item 2 of first sheet)**

This international search report has not been established in respect of certain claims under Article 17(2)(a) for the following reasons:

1. ☑  Claims Nos.: **1-7**
    because they relate to subject matter not required to be searched by this Authority, namely:

    [1]   Claims 1-7 relate to a use for annexin A5 in preventing and/or treating a disease or a condition,
          and thus belong to subject matter for which the International Searching Authority is not required to
          perform a search under PCT Rule 39.1(iv). The present search has been performed on the basis of a
          use of annexin A5 in preparing a drug for preventing and/or treating a disease or a condition.

2. ☐  Claims Nos.:
    because they relate to parts of the international application that do not comply with the prescribed requirements to such an
    extent that no meaningful international search can be carried out, specifically:

3. ☐  Claims Nos.:
    because they are dependent claims and are not drafted in accordance with the second and third sentences of Rule 6.4(a).

Form PCT/ISA/210 (continuation of first sheet) (January 2015)

| Patent document cited in search report | | | Publication date (day/month/year) | Patent family member(s) | | | Publication date (day/month/year) |
|---|---|---|---|---|---|---|---|
| CN | 107921068 | A | 17 April 2018 | WO | 2016209057 | A3 | 16 February 2017 |
| | | | | AU | 2016283612 | A1 | 15 February 2018 |
| | | | | KR | 101830945 | B1 | 23 February 2018 |
| | | | | US | 10688135 | B2 | 23 June 2020 |
| | | | | JP | 2018518503 | A | 12 July 2018 |
| | | | | EP | 3315133 | A2 | 02 May 2018 |
| | | | | CA | 2990807 | A1 | 29 December 2016 |
| | | | | EP | 3315133 | A4 | 30 May 2018 |
| | | | | US | 2018228845 | A1 | 16 August 2018 |
| | | | | AU | 2016283612 | B2 | 24 January 2019 |
| | | | | EP | 3315133 | B1 | 22 July 2020 |
| | | | | KR | 20170001943 | A | 05 January 2017 |
| | | | | WO | 2016209057 | A2 | 29 December 2016 |
| CN | 106714814 | A | 24 May 2017 | JP | 2017520578 | A | 27 July 2017 |
| | | | | KR | 101675123 | B1 | 14 November 2016 |
| | | | | EP | 3162372 | A1 | 03 May 2017 |
| | | | | EP | 3162372 | A4 | 13 June 2018 |
| | | | | WO | 2015199499 | A1 | 30 December 2015 |
| | | | | EP | 3403658 | A1 | 21 November 2018 |
| | | | | US | 2017136068 | A1 | 18 May 2017 |
| | | | | KR | 20160001908 | A | 07 January 2016 |
| | | | | JP | 6400744 | B2 | 03 October 2018 |
| CN | 101405017 | A | 08 April 2009 | US | 2006258584 | A1 | 16 November 2006 |
| | | | | CA | 2608070 | A1 | 14 September 2006 |
| | | | | US | 2008280831 | A1 | 13 November 2008 |
| | | | | AU | 2006220513 | A1 | 14 September 2006 |
| | | | | EP | 1877437 | A2 | 16 January 2008 |
| | | | | US | 7393833 | B2 | 01 July 2008 |
| | | | | US | 7807644 | B2 | 05 October 2010 |
| | | | | EP | 1877437 | A4 | 22 July 2009 |
| | | | | WO | 2006096828 | A2 | 14 September 2006 |
| | | | | WO | 2006096828 | A3 | 20 November 2008 |
| CN | 102105594 | A | 22 June 2011 | EP | 2321426 | A4 | 14 December 2011 |
| | | | | US | 8999661 | B2 | 07 April 2015 |
| | | | | EP | 2321426 | A1 | 18 May 2011 |
| | | | | WO | 2010014222 | A1 | 04 February 2010 |
| | | | | US | 2010034800 | A1 | 11 February 2010 |
| WO | 2012136819 | A1 | 11 October 2012 | EP | 2694538 | A1 | 12 February 2014 |
| | | | | US | 2020179484 | A1 | 11 June 2020 |
| | | | | US | 2018015142 | A1 | 18 January 2018 |
| | | | | US | 2014037614 | A1 | 06 February 2014 |
| | | | | ES | 2607066 | T3 | 29 March 2017 |
| | | | | EP | 2694538 | B1 | 14 September 2016 |
| | | | | DK | 2694538 | T3 | 02 January 2017 |
| | | | | HU | E030922 | T2 | 28 June 2017 |
| | | | | PL | 2694538 | T3 | 28 April 2017 |

Form PCT/ISA/210 (patent family annex) (January 2015)

## REFERENCES CITED IN THE DESCRIPTION

**Patent documents cited in the description**

- CN 201910815496

**Non-patent literature cited in the description**

- *J. Mol. Biol.,* 1992, vol. 223 (3), 683-704
- JOLKKONEN J ; GALLAGHER NP ; ZILLES K ; SIVENIUS J. *Behav Brain Res,* 2003, vol. 138, 187-200
- LONGA EZ ; WEINSTEIN PR ; CARLSON S ; CUMMINS R. *Stroke,* 1989, vol. 20, 84-91
- BEDERSON JB ; PITTS LH ; TSUJI M ; NISHIMURA MC ; DAVIS RL ; BARTKOWSKI H. *Stroke,* 1986, vol. 17, 472-476
- BEDERSON JB ; PITTS LH ; GERMANO SM ; NISHIMURA MC ; DAVIS RL ; BARTKOWSKI HM. *Stroke,* 1986, vol. 17, 1304-8
- YANG Y ; SHUAIB A ; LI Q. *JNeurosci Methods,* 1998, vol. 84, 9-16